# EUROPEAN PATENT SPECIFICATION

(11) **EP 3 924 978 B1**
(45) Date of publication and mention of the grant of the patent: **31.07.2024**
(21) Application number: 20709325.3
(22) Date of filing: 14.02.2020
(51) Int. Cl.: G16H 20/30, A61B 5/11

(54) **SYSTEM AND METHOD FOR DETECTING AND EVALUATING RISK FACTORS FOR THE PROTECTION OF THE HEALTH OF WORKERS**
SYSTEM UND VERFAHREN ZUR ERFASSUNG UND BEWERTUNG VON RISIKOFAKTOREN FÜR DEN GESUNDHEITSSCHUTZ VON ARBEITERN
SYSTÈME ET PROCÉDÉ DE DÉTECTION ET D'ÉVALUATION DE FACTEURS DE RISQUE DESTINÉS À LA PROTECTION DE LA SANTÉ DES TRAVAILLEURS

(30) Priority: 15.02.2019 IT 201900002215
(43) Date of publication of application: 22.12.2021
(73) Proprietor: Integra Srl, 88100 Catanzaro (CZ) (IT)
(72) Inventor: RUBINO, Giuseppe, 88100 Catanzaro (CZ) (IT)
(74) Representative: Giuliano, Natalia
(86) International application number: PCT/IB2020/051257
(87) International publication number: WO 2020/165854

(56) References cited:
- EP-A1- 2 801 318
- US-A1- 2007 250 286
- US-A1- 2013 217 352
- US-A1- 2014 266 737
- US-A1- 2017 245 806

## Description

The present invention relates to a system for detecting and evaluating risk factors for the protection of the health of workers.

The present invention relates also to a method for detecting and evaluating risk factors for the protection of the health of workers.

In particular, the present invention relates to a system and a method for detecting and evaluating the risk factors for the protection of the health of workers, of the type comprising wearable devices able to gather data, and specific software programs able to analyze and process such data.

As it is known, the continuous occurrences of accidents and more or less debilitating pathologies related to the usual and continued execution of work routines increasingly puts the emphasis on safety, in order to protect the health of workers, with particular attention to the sectors most at risk.

At the same time, the existence of increasingly stringent regulations related to the safety in the workplace, and the technical progress that allows the creation of smart devices more and more advanced and miniaturized, contribute to implementing procedures accompanied by technologies designed to verify the health of the workforce and to reduce the risk for chronic diseases, in particular for the musculoskeletal system, to occur.

CHECK-LIST, CTD INDEX, ERGONOMIC STRESS INDEX, OCRA INDEX, OWAS, RULA e STRAIN INDEX are some of the most innovative models and methods by which it is possible to evaluate the risk related to repetitive and demanding activities in the workplace.

On the basis of the statistical data available on work pathologies, the following stand out, each with its own percentage incidence: backache (30% of cases); stress (26%); upper limb pain (12%); lower limb pain (11%); neck and shoulder pain (210).

Biomechanical overload to the spine, for example, stands out among the complications with the highest risk of onset in the workplace.

By biomechanical overload is to be understood an event that leads to a stress on one or more anatomical structures (in the first instance joints, bones, muscles and tendons) that do not work in their normal physiological environment or that in any case exceed the characteristics for which they were "designed".

Specifically, reference is made to the repetitive recurrent tasks, present in the activities in which the worker performs the same sequence of actions, and therefore to a series of movements characterized by a beginning and an end, which occur cyclically.

Repetitiveness of certain actions, incongruous postures, lifting and shifting of loads carried out incorrectly, are the main factors that in the long term contribute to causing physiological damages to the musculoskeletal system.

The Title VI of the Italian Law 81/2008, addressing health and safety in the workplace, even if not directly, mentions activities involving repetitive movements of the upper limbs. The high-frequency manipulation of instruments with a low weight, for example, is believed to be a particular type of manual handling of loads and can be the cause of the onset of several pathologies.

Among the various complications it is possible to mention arthrosis beaks, hernias, lumbago and also deviations from the normal curves of the spine both along the front and the sagittal axis (in profile).

Scapulohumeral periarthritis, epicondylitis, carpal tunnel syndrome, tendinitis and meniscus injuries are other diseases that can affect the health of both lower and upper limbs.

Therefore, it is important both to monitor the execution of certain actions in the workplace to fix any error and detect possible physical limits (either inborn or resulting from previous traumas, or even related to insufficient strength) which would represent unsuitability for the task to be performed and which would lead to high probability over time of the onset of diseases with consequences also disabling, and to train each individual worker, also through specific training courses, to execute in the right way more or less demanding repetitive working activities.

Several systems for detecting and evaluating risk factors for the protection of the health of workers are currently known.

The international application WO2008038025A1, for example, describes a method and a system which allow healthcare and working operating conditions for personnel of an organization to be monitored and, when necessary, taken into account in the allocation of work tasks for said personnel to perform on behalf of the organization. The invention is particularly of use for an organization employing a number of different personnel who may change in identity relatively frequently and/or frequently move locations as they perform working tasks. The system may also be used to track the health of a person to help the organization meet health and safety requirements.

The US utility patent application US2007250286A1 describes a motion analysis system in which sensor elements attached to movable body segments record movement parameters including angular velocity and acceleration. A control device receives the movement parameters and determines an overall motion of the movable body segments. The overall motion is analyzed against an acceptable motion model to determine whether the overall motion is within acceptable limits. The sensor elements and control device are lightweight and can be worn during normal movement activities thereby allowing monitoring of work-based activities, such as lifting or typing. The invention is useful for detecting and correcting problems leading to lower back disorders and repetitive strain injuries.

In addition, the US patent application US2013217352A1 discloses a miniaturized, ruggedized, field-deployable Portable Exposure Assessment System (PEAS)used to remotely monitor workers and provide real-time warning of exposure to musculoskeletal injury conditions via alarm and smart-phone transmission. The PEAS unit wirelessly acquires exposure data from sensors, conducts initial data analysis, triggers proximal and remote alarms, sends out text messages with abnormal data, GPS locations, and time stamps to a safety office; and saves data for more extensive assessment. Sensor technology is used in this field-deployable system to simultaneously measure and collect the body loads and awkward postures imposed by package handling as well as driving-related, low-frequency vibration exposures.

The US utility patent US5163440, still, discloses a method for analyzing muscle function including the steps of: (a) locating in a human control subject a predetermined muscle group activatable to produce a given torque; (b) fixing over each of a plurality of individual muscles in the group an electrode adapted to detect myoelectric signals generated therein; (c) activating of the muscles to generate myoelectric signals therein; (d) processing the myoelectric signals to determine a plurality of predetermined values thereof; (e) repeating steps (a), (b), (c) and (d) for each of a plurality of other control subjects; (f) determining a normative range of the predetermined values determined in steps (d) and (e); (g) repeating steps (a), (b), (c) and (d) for a human test subject; and (h) comparing the predetermined values determined in step (g) with the normative range determined in step (f). Dysfunction in the muscle group of the test subject can be determined by comparison of the processed myoelectric signal values.

Finally, the US patent application US2018793A1 in the name of Microsoft Technology Licensing LLC describes examples correlating health data outcomes to working data of a user, recommending future working activities based upon the aforementioned correlation. One example provides a computing device configured to monitor health data related to health behavior of a user over time, determine a baseline value for a health outcome of the user based on the monitored health data, and receive working data relating to a work schedule of the user. The computing device is configured to determine that the deviation in the health outcome correlates with the working data, and based at least upon determining that the deviation in the health outcome correlates with the working data, output a notification of the determination to the user.

However, the currently known systems for detecting and evaluating risk factors for the protection of the health of workers suffer from intrinsic limitations such as the absence of wearable components provided with sensors that detect and sample the physical parameters of any single worker, supporting the planning and the management of working conditions in order to avoid the onset of diseases affecting the musculoskeletal system.

A further limitation of the known systems for detecting and assessing risk factors for the protection of the health of workers is the lack of advanced IT tools that analyze the amount of data collected in order to highlight and report the situations with potential risk of onset of the aforementioned diseases.

The purpose of the present invention is to provide a system for detecting and assessing risk factors for the protection of the health of workers that allows to detect the occurrences of the situations of potential risk of onset of diseases having, therefore, characteristics that overcome the limits that still affect the current system for detecting and assessing risk factors for the protection of the health of workers, with reference to the known technique.

A further purpose of the present invention is to provide a method for detecting and assessing risk factors for the protection of the health of workers.

Finally, another purpose of the present invention is to provide a system and a method for detecting and assessing risk factors for the protection of the health of workers, aimed at the rehabilitation of workers or at determining the need to start a rehabilitation phase.

According to the present invention, a system for detecting and assessing risk factors for the protection of the health of workers is provided, as defined in claim 1.

According to the present invention, a method for detecting and assessing risk factors for the protection of the health of workers is also provided, as defined in claim 11.

For a better understanding of the present invention, a preferred embodiment is now described, purely by way of nonlimiting example, with reference to the annexed drawings, in which:
- figure 1 shows an overall schematic view of a system for detecting and assessing risk factors for the protection of the health of workers, according to the invention;
- figure 2 shows a block diagram of the steps of a method for detecting and assessing risk factors for the protection of the health of workers, according to the invention.

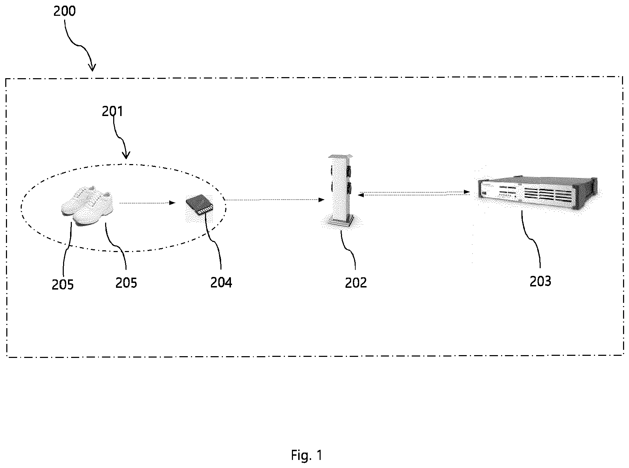

With reference to such figures and, in particular, to figure 1, a system for detecting and assessing risk factors for the protection of the health of workers is shown, according to the invention.

In particular, the system 200 for detecting and assessing risk factors for the protection of the health of workers comprises:
- at least one device 201 able to be worn by a worker comprising a rewritable memory device 204 and at least one sensor able to detect physical parameters related to a working activity of the worker;
- at least one data collecting control unit 202 configured for data transmission from the at least one device 201, provided with connectivity to a corporate LAN network and to the Internet;
- a central processing unit 203 provided with connectivity to a corporate LAN network and to the Internet, configured for data transmission to and from the at least one data collecting control unit 202.

According to an aspect of the invention, the at least one device 201 is configured to sample and temporarily save to the rewritable memory device 204 data related to the physical parameters concerning the working activity of the worker when they exceed first set threshold values.

In use, advantageously according to the invention, the system 200 allows to evaluate the occurrence of conditions potentially at risk for the musculoskeletal system of the worker, according to limit values set and associated with the physical parameters and stored through said first set threshold values.

The at least one device 201 consists of a pair of shoes, each of them comprising a programmable logic unit mounted on an electronic board and configured to correlate the data related to the physical parameters with the data related to the physical parameters concerning the other shoe.

According to an aspect of the invention, the at least one sensor is a load cell, for example a sensor that uses miniaturized electronic strain gauges, and a detector of the relative position of each shoe, such as a displacement sensor, able to measure physical parameters, i.e., load variations, the posture and the position of the center of gravity of the body of the worker, said load cell and detector being connected to the electronic board.

According to an aspect of the invention, the electronic board is comprised in an insole 205, with both shoes comprising their own insole 205.

The system 200 allows to evaluate the posture conditions, the balance and the distribution of the load to which the worker is subject.

According to another aspect of the invention, the at least one device 201 is configured to temporarily detect and save to the rewritable memory device 204 the frequency with which the detected physical parameters reach second set threshold values.

According to an aspect of the invention, the load variations detected by both shoes are mutually correlated by means of the programmable logic unit, or PLU, mounted on the electronic board comprised in the shoe, preferably in the insole 205.

Advantageously according to the invention, by determining the position of the center of gravity of the worker measured through the at least one sensor, the system 200 allows to detect the vertical projection of such parameter coming out of a "circle of balance", delimiting a potential risky condition for the worker.

According to the invention, the system 200, by correlating detections coming from both shoes worn by the worker, by means of the respective programmable logic unit, is able to detect conditions potentially risky for the health of the worker and dangerous behaviors, excluding situations that would be otherwise regarded as risky if depending on each single shoe, in the absence of the correlation of the two measurements.

In use, advantageously according to the invention, the correlation of the data sampled starting from the detection of the physical parameters operated by the at least one sensor allows to operate a smart filtering of potentially risky working activities, avoiding an excessive number of warnings generated by the system 200, as described in the following.

According to another aspect of the invention, the at least one sensor comprised in the at least one device 201, suitable for detecting the physical parameters characterizing repetitive working activities, is connected to the electronic board, for example a flexible PCB, comprising both the rewritable memory device 204, a short-range wireless connection module (Bluetooth LE or NFC) and the programmable logic unit, for example a microcontroller provided with a specific integrated development environment (IDE) .

The at least one device 201 can integrate a second array of sensors positioned close to the heel, able to evaluate the posture conditions and the position of the center of gravity of the body of the worker.

In use therefore, advantageously, the system 200 is able to detect physical parameters characterizing recurrent activities such as repeated lifting of moderate loads, incongruous postures, and to attest reaching and exceeding said second set threshold values, related to the limit number of repetitions in a given period of time, for example during the typical working day, that in the long term could generate diseases affecting the musculoskeletal system.

According to an aspect of the invention, the at least one device 201 is configured to transmit the data related to the physical parameters and temporarily saved to the rewritable memory device 204 to the at least one data collecting control unit 202 through a short-range wireless connection, such as Bluetooth or NFC.

According to an aspect of the invention, the at least one data collecting control unit 202, for example an electronic column such as a totem in which a specific electronic unit including a wireless communication module is mounted, is configured for the periodic storage of the data related to the physical parameters temporarily detected and saved by the at least one device 201 and for the periodic sending of such data to the central processing unit 203 through the corporate LAN network or the Internet.

Advantageously according to the invention, the programmable logic unit mounted on the electronic board installed in the at least one device 201 is configured to erase the data related to the physical parameters saved to the rewritable memory device 204, following the periodic transfer of said data to the at least one data collecting control unit 202, so as to avoid the repeated transmission, i.e. the duplicated transmission, of the same data.

According to another aspect of the invention, the data related to the physical parameters detected by the at least one device 201 are samples of said parameters acquired according to a sampling frequency given by the logic executed by the aforementioned programmable logic unit.

A further advantage granted by the use of a programmable logic unit is the implementation of tools for alerting and monitoring the working activity of the worker in real-time.

According to another aspect of the invention, the at least one device 201 comprises RFID tags able to uniquely identify the worker.

In this way, advantageously, the system 200 allows to regroup in an orderly and specific manner, for each worker, the physical parameters sampled and sent by means of the data collecting control unit 202 and the corporate LAN network or the Internet to the central processing unit 203.

According to an aspect of the invention, the at least one device 201 periodically sends the data related to the physical parameters to a smartphone of the worker, such a smartphone sending the same data related to the physical parameters to the central processing unit 203.

According to an aspect of the invention, the central processing unit 203 is a local server or a cloud server, provided with both a database configured for storing the data related to the physical parameters detected by the at least one device 201 and the frequency with which the detected physical parameters reach second set threshold values, and a software program configured to analyze such data.

In this way, advantageously, the system 200 allows to individually profile every single worker and to draw up for each of said worker a digital record aimed at suggesting corrections of erroneous behaviors during repetitive working activities, in order to address all aspect related to OSH (Occupational Safety and Health).

According to another aspect of the invention, the software program installed on the central processing unit 203 is configured for sending periodic reports to a predefined user, said reports containing the data related to the physical parameters saved to the rewritable memory device 204 and to the frequency with which the physical parameters detected reach second set threshold values concerning each worker.

Advantageously according to the invention, the system 200, by means of the periodic reports containing the recognition, the frequency and the duration of the harsh conditions to which the worker is subject, in particular recurrent and repetitive activities related to the handling of loads or incongruous postures, allows to pursue the most appropriate conduct guidelines to be adopted in the workplace, to be imparted also through suitable training courses for the workers.

A further advantage of the system 200 according to the invention is that it allows to evaluate the health risk of worker concerning each detected, saved and analyzed event.

During the analysis of the data detected by the at least one device 201, concerning the physical parameters related to the working activity of the worker, the software running on the central processing unit 203 compares such data with those saved to a preloaded table containing the mapping, for each activity executed by the worker and examined by the system 200, of the first and second set threshold values concerning each monitored activity.

In this way the system 200 implements a comparative and personalized analysis followed by the compilation and the transmission of the reports of each single worker, said reports containing the events related to reaching and exceeding the aforementioned first and second set threshold values.

Said threshold values, for example the maximum liftable weight or the maximum tolerable number of moderate weights raised/shifted, are set by the system on the basis of a preliminary calibration phase which also takes into account the physiological and personal variables of the worker, e.g., age, weight, presence of pathologies and previous significant clinical events.

According to another aspect of the invention, the system 200 is configured to detect the physical parameters related to the activity of the worker following an injury.

In this way, advantageously, the at least one device 201 allows to evaluate quality and timing of the physical recovery of the worker following an event that has temporarily impaired his motor functions. The system 200, in this case, is applied to the monitoring of physical parameters aimed at the rehabilitation of the workers or at determining the need to start a rehabilitation phase.

According to another embodiment not forming part of the invention, not shown in the figures, the at least one device is a device provided with a tilt sensor and is able to be fixed to the back, to the hand, to the elbow, to the neck or to the knee of the worker.

According to another aspect not forming part of the invention, the at least one device is a glove equipped with a tilt sensor and is able to be worn over a hand of the worker in order to detect repetitive efforts or movements executed by the wrist.

Even in these further embodiments, the at least one device comprises at least one sensor able to detect physical parameters related to a working activity of the worker, being said sensor mounted or connected to an electronic board placed in the device and having a programmable logic unit, or PLU, configured to sample data related to the aforementioned physical parameters. The data, sampled by the PLU, are saved to a rewritable memory device and here erased after being sent to a data collecting control unit and, again, to a central processing unit for further processing.

According to these two further embodiments not forming part of the invention, advantageously, the system for detecting and assessing risk factors for the protection of the health of workers is able to detect physical parameters characterizing recurrent activities such as a repetitive bending of the back, incongruous postures at the desk and in movement, the number of twists of the wrist, and to assess the reaching and exceeding said second set threshold values, related to the limit number of repetitions in a given period of time, for example during the typical working day, that in the long term could generate diseases affecting the musculoskeletal system.

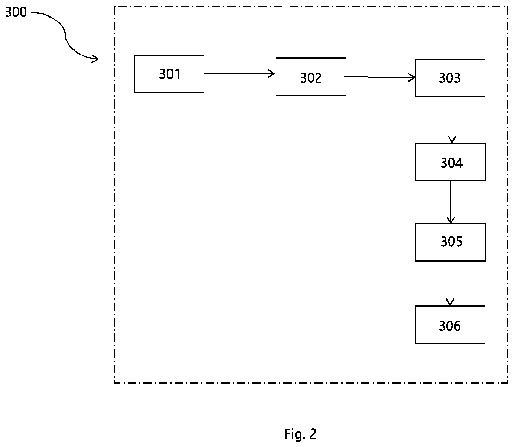

As previously said and shown in figure 2, the present invention relates also to a method 300 for detecting and evaluating risk factors for the protection of the health of workers by using the system 200, comprising the steps:
- 301 of detecting physical parameters concerning a working activity of a worker by means of at least one device able to be worn by said worker and equipped with at least one sensor;
- 302 of saving data related to the physical parameters to a rewritable memory device by means of a programmable logic unit mounted on an electronic board comprised in the at least one device;
- 303 of sending the data related to the physical parameters to a data collecting control unit through a short-range wireless connection and of erasing said data from the rewritable memory device following said sending;
- 304 of sending the data related to the physical parameters concerning the working activity of the worker to a central processing unit via a corporate LAN network or the Internet;
- 305 of processing the data related to the physical parameters by comparing said data with first and second set threshold values, said threshold values being set for each physical parameter evaluated;
- 306 of compiling recurring reports following the step 305 of processing the data related to the physical parameters and of sending said recurring reports to a default user.

Therefore, the system and the method for detecting and evaluating risk factors for the protection of the health of workers according to the invention represent an effective tool to analyze situations and conditions involving a relevant risk of occurrence of chronic diseases affecting the musculoskeletal system.

A further advantage of the system for detecting and evaluating risk factors for the protection of the health of workers according to the invention is that is safe.

Still, the system for detecting and evaluating risk factors for the protection of the health of workers according to the invention is of easy and comfortable use.

Finally, the system for detecting and evaluating risk factors for the protection of the health of workers according to the invention is inexpensive.

It is finally clear that the system and the method for detecting and evaluating risk factors for the protection of the health of workers described and illustrated herein can be subject to modifications and variations without departing from the protective scope of the present invention, as defined in the appended claims.

## Claims

1. System (200) for detecting and evaluating risk factors for the protection of the health of workers, comprising:
- at least one device (201) able to be worn by a worker and consisting of a pair of shoes, each shoe comprising a rewritable memory device (204) and at least one sensor able to detect physical parameters related to a working activity of the worker, said at least one device (201) configured to sample and temporarily save to the rewritable memory device (204) data related to the physical parameters when they exceed first set threshold values;
- at least one data collecting control unit (202) configured for data transmission from the at least one device (201), provided with connectivity to a corporate LAN network and to the Internet;
- a central processing unit (203) provided with connectivity to a corporate LAN network and to the Internet, configured for data transmission to and from the at least one data collecting control unit (202);
**characterized in that** the at least one sensor is a load cell and a detector of the relative position of each shoe able to measure the posture and the position of the center of gravity of the body of said worker and to evaluate the posture conditions, the balance and the distribution of the load to which the worker is subject, and said sensor being connected to an electronic board, and **in that** each of said shoes comprises a programmable logic unit mounted on the electronic board and configured to correlate the data related to the physical parameters with the data related to the physical parameters concerning the other shoe, so as to detect conditions potentially risky for the health of the worker excluding situations otherwise regarded as risky if depending on each single shoe.

2. System (200) according to claim 1, **characterized in that** the electronic board is comprised in an insole (205) inserted in the shoe.

3. System (200) according to claim 1, **characterized in that** said at least one device (201) is configured to detect and temporarily save to the rewritable memory device (204) the frequency with which the detected physical parameters reach second set threshold values.

4. System (200) according to claim 1, **characterized in that** the at least one device (201) is configured to transmit the data related to the physical parameters to the at least one data collecting control unit (202) through a short-range wireless connection.

5. System (200) according to claim 1, **characterized in that** the at least one data collecting control unit (202) is configured for the periodic saving of the data related to the physical parameters detected and temporarily stored by the at least one device (201) and for the periodic sending of the data to the central processing unit (203) through the corporate LAN network or the Internet.

6. System (200) according to claim 1, **characterized in that** the programmable logic unit mounted on the electronic board is configured to erase the data related to the physical parameters saved to the rewritable memory device (204) following the transmission of said data to the at least one data collecting control unit (202).

7. System (200) according to claim 1, **characterized in that** the at least one device (201) comprises RFID tags able to uniquely identify the worker.

8. System (200) according to claim 1, **characterized in that** the central processing unit (203) is a local server or a cloud server which hosts a database configured for storing the data related to the physical parameters detected by the at least one device (201) and to the frequency with which the detected physical parameters reach second set threshold values, and a software program configured for analyzing such data.

9. System (200) according to claim 8, **characterized in that** the software program installed on the central processing unit (203) is configured for sending recurring reports to a default user, said reports containing data related to physical parameters saved to the rewritable memory device (204) and to the frequency with which the detected physical parameters reach second set threshold values concerning each worker.

10. Method (300) of use of the system according to one of the previous claims for detecting and evaluating risk factors for the protection of the health of workers comprising the steps:
- (301) of detecting physical parameters concerning a working activity of a worker by means of the at least one device able to be worn by a worker and equipped with the at least one sensor;
- (302) of saving data related to the physical parameters to the rewritable memory device by means of a programmable logic unit mounted on the electronic board comprised in the at least one device;
- (303) of sending the data related to the physical parameters to the data collecting control unit through a short-range wireless connection and of erasing said data from the rewritable memory device following said sending;
- (304) of sending the data related to the physical parameters concerning the working activity of the worker to the central processing unit through a corporate LAN network or the Internet;
- (305) of processing the data related to the physical parameters by comparing said data with first and second set threshold values, said threshold values being set for each physical parameter evaluated;
**characterized in** comprising the step (306) of compiling recurring reports following the step (305) of processing the data related to the physical parameters and of sending said recurring reports to a default user.

## Patentansprüche

1. System (200) zum Erfassen und Bewerten von Risikofaktoren für den Arbeitsschutz, das Folgendes umfasst:
- mindestens ein Gerät (201), das von einem Mitarbeiter getragen werden kann und aus einem Paar Schuhe besteht, wobei jeder Schuh ein wiederbeschreibbares Speichergerät (204) sowie mindestens einen Sensor umfasst, der physikalische Parameter in Bezug auf die Arbeitstätigkeit des Mitarbeiters erfassen kann, und wobei das mindestens eine Gerät (201) so konfiguriert ist, dass es Daten in Bezug auf die physikalischen Parameter abtastet und vorübergehend auf dem wiederbeschreibbaren Speichergerät (204) speichert, wenn diese erste festgelegte Schwellenwerte überschreiten;
- mindestens eine Datenerfassungs-Steuerungseinheit (202), die für das Übertragen der Daten vom mindestens einen Gerät (201) konfiguriert ist und mit einem Unternehmens-LAN-Netzwerk und dem Internet verbunden ist;
- eine zentrale Verarbeitungseinheit (203), die mit einem Unternehmens-LAN-Netzwerk und dem Internet verbunden ist und für die Datenübertragung zu und von der mindestens einen Datenerfassungs-Steuerungseinheit (202) konfiguriert ist;
**dadurch gekennzeichnet, dass** es sich beim mindestens einen Sensor um einen Druckmesser und einen Detektor für die relative Position der einzelnen Schuhe handelt, der die Körperhaltung und die Position des Körperschwerpunkts des Mitarbeiters messen und die Körperhaltungsgegebenheiten, das Gleichgewicht und die Verteilung der auf den Mitarbeiter einwirkenden Last auswerten kann, wobei der Sensor mit einer elektronischen Platine verbunden ist, und wobei jeder Schuh eine programmierbare Logikeinheit umfasst, die auf der elektronischen Platine angebracht und so konfiguriert ist, dass sie die Daten, die sich auf die physikalischen Parameter beziehen, mit den Daten, die sich auf die physikalischen Parameter des anderen Schuhs beziehen, korreliert, um Zustände zu erkennen, die für die Gesundheit des Mitarbeiters potenziell riskant sind, und wobei Situationen ausgeschlossen werden, die anderweitig als riskant gelten, wenn sie sich auf jeden einzelnen Schuh beziehen.

2. System (200) gemäß Anspruch 1, dass sich dadurch auszeichnet, dass sich die elektronische Platine in einer in den Schuh eingesetzten Einlegesohle (205) befindet.

3. System (200) gemäß Anspruch 1, das sich dadurch auszeichnet, dass das mindestens eine Gerät (201) so konfiguriert ist, dass es die Häufigkeit, mit der die erfassten physikalischen Parameter zweite festgelegte Schwellenwerte erreichen, erfasst und vorübergehend auf dem wiederbeschreibbaren Speichergerät (204) speichert.

4. System (200) gemäß Anspruch 1, das sich dadurch auszeichnet, dass das mindestens eine Gerät (201) so konfiguriert ist, dass es die auf die physikalischen Parameter bezogenen Daten über eine drahtlose Kurzstreckenverbindung an die mindestens eine Datenerfassungs-Steuerungseinheit (202) überträgt.

5. System (200) gemäß Anspruch 1, das sich dadurch auszeichnet, dass die mindestens eine Datenerfassungs-Steuerungseinheit (202) so konfiguriert ist, dass sie die Daten, die sich auf die vom mindestens einen Gerät (201) erfassten und vorübergehend gespeicherten physikalischen Parameter beziehen, regelmäßig speichert und über das Unternehmens-LAN-Netzwerk oder das Internet an die zentrale Verarbeitungseinheit (203) sendet.

6. System (200) gemäß Anspruch 1, das sich dadurch auszeichnet, dass die auf der elektronischen Platine montierte programmierbare Logikeinheit so konfiguriert ist, dass sie die auf die physikalischen Parameter bezogenen und auf dem wiederbeschreibbaren Speichergerät (204) gespeicherten Daten nach dem Übertragen an die mindestens eine Datenerfassungs-Steuerungseinheit (202) löscht.

7. System (200) gemäß Anspruch 1, das sich dadurch auszeichnet, dass das mindestens eine Gerät (201) RFID-Etiketten umfasst, mit denen der Mitarbeiter eindeutig identifiziert werden kann.

8. System (200) gemäß Anspruch 1, das sich dadurch auszeichnet, dass es sich bei der zentralen Verarbeitungseinheit (203) um einen lokalen Server oder einen Cloud-Server handelt, auf dem sich eine Datenbank befindet, in der die sich auf die vom mindestens einen Gerät (201) erfassten physikalischen Parameter und auf die Häufigkeit, mit der die erfassten physikalischen Parameter zweite festgelegte Schwellenwerte erreichen, beziehenden Daten gespeichert werden, und wobei zudem ein Softwareprogramm vorhanden ist, das zum Analysieren der entsprechenden Daten konfiguriert ist.

9. System (200) gemäß Anspruch 8, das sich dadurch auszeichnet, dass das auf der zentralen Verarbeitungseinheit (203) installierte Softwareprogramm so konfiguriert ist, dass es wiederkehrende Berichte an einen Standardbenutzer sendet, wobei die Berichte Daten enthalten, die sich auf die auf dem wiederbeschreibbaren Speichergerät (204) gespeicherten physikalischen Parameter sowie auf die Häufigkeit beziehen, mit der die erfassten physikalischen Parameter für die einzelnen Mitarbeiter zweite festgelegte Schwellenwerte erreichen.

10. Methode (300) zum Verwenden des Systems gemäß einem der vorherigen Ansprüche zum Erfassen und Bewerten von Risikofaktoren für den Schutz der Gesundheit von Mitarbeitern, die die folgenden Schritte umfasst:
- (301) Erfassen von physikalischen Parametern, die die Arbeitstätigkeit des Mitarbeiters betreffen, mithilfe des mindestens einen Geräts, das vom Mitarbeiter getragen werden kann und mit dem mindestens einen Sensor ausgestattet ist;
- (302) Speichern von Daten, die sich auf die physikalischen Parameter beziehen, auf dem wiederbeschreibbaren Speichergerät mithilfe einer programmierbaren Logikeinheit, die auf der elektronischen Platine des mindestens einen Geräts angebracht ist;
- (303) Senden der auf die physikalischen Parameter bezogenen Daten an die Datenerfassungs-Steuerungseinheit über eine drahtlose Kurzstreckenverbindung und Löschen der Daten vom wiederbeschreibbaren Speichergerät im Anschluss an das dem Senden;
- (304) Senden der auf die physikalischen Parameter bezogenen Daten, die die Arbeitsaktivität des Mitarbeiters betreffen, an die zentrale Verarbeitungseinheit über ein Unternehmens-LAN-Netzwerk oder das Internet;
- (305) Verarbeiten der auf die physikalischen Parameter bezogenen Daten durch Vergleichen dieser Daten mit ersten und zweiten festgelegten Schwellenwerten, wobei die Schwellenwerte jeweils für die einzelnen ausgewerteten physikalischen Parameter festgelegt werden;
**dadurch gekennzeichnet, dass** dies den Schritt (306) des Zusammenstellens von wiederkehrenden Berichten im Anschluss an Schritt (305) der Verarbeitung der auf die physikalischen Parameter bezogenen Daten und des Sendens der wiederkehrenden Berichte an einen Standardbenutzer umfasst.

## Revendications

1. Système (200) pour détecter et évaluer les facteurs de risque pour la protection de la santé des travailleurs, comprenant :
- au moins un dispositif (201) pouvant être porté par un travailleur et composé d'une paire de chaussures, chaque chaussure comprenant un dispositif de mémoire réinscriptible (204) et au moins un capteur capable de détecter des paramètres physiques liés à l'activité professionnelle du travailleur, ce dispositif au moins unique (201) est configuré pour échantillonner et sauvegarder temporairement sur le dispositif de mémoire réinscriptible (204) des données liées aux paramètres physiques lorsqu'ils commencent à dépasser les valeurs limites ;
- au moins une unité de contrôle collectant des données (202) configurée pour la transmission de données à partir d'au moins un dispositif (201), dotée d'une connexion à un réseau LAN d'entreprise et à internet ;
- une unité centrale de traitement (203) dotée d'une connexion à un réseau LAN d'entreprise et à internet, configurée pour la transmission de données vers et à partir de l'unité de contrôle au moins unique collectant des données (202);
**caractérisée par le fait que** le capteur au moins unique est une cellule de charge et un détecteur de la position relative de chaque chaussure du travailleur, capable de mesurer la posture et la position du centre de gravité de ce travailleur et d'évaluer les conditions de posture, l'équilibre et la répartition de la charge à laquelle le travailleur est soumis, ce capteur étant connecté à une carte électronique, et en ce que chacune de ces chaussures comprend une unité logique programmable installée sur la carte électronique et configurée pour corréler les données liées aux paramètres physiques concernant l'autre chaussure, de manière à détecter des conditions pouvant représenter un risque pour la santé du travaillant, en excluant les situations autrement considérées comme risquées si elles dépendent de chaque chaussure.

2. Système (200) selon la réclamation 1, **caractérisé par le fait que** la carte électronique est composée d'une semelle intérieure (205) insérée dans la chaussure.

3. Système (200) selon la réclamation 1, **caractérisé par le fait que** le dispositif au moins unique (201) est configuré pour détecter et sauvegarder temporairement sur le dispositif de mémoire réinscriptible (204) la fréquence à laquelle les paramètres physiques détectés atteignent les deuxièmes valeurs limites fixées.

4. Système (200) selon la réclamation 1, **caractérisé par le fait que** le dispositif au moins unique (201) est configuré pour transmettre les données liées aux paramètres physiques à l'unité de contrôle au moins unique collectant les données (202) grâce à une connexion sans fil de courte portée.

5. Système (200) selon la réclamation 1, **caractérisé par le fait que** l'unité de contrôle au moins unique collectant les données (202) est configurée pour la sauvegarde périodique des données liées aux paramètres physiques détectés et temporairement stockées par le dispositif au moins unique (201) et pour la transmission périodique des données vers l'unité centrale de traitement (203) via le réseau LAN d'entreprise ou internet.

6. Système (200) selon la réclamation 1, **caractérisé par le fait que** l'unité logique programmable installée sur la carte électronique est configurée pour effacer les données liées aux paramètres physiques sauvegardées sur le dispositif de mémoire réinscriptible (204) à la suite de la transmission de ces données vers l'unité de contrôle au moins unique collectant les données (202).

7. Système (200) selon la réclamation 1, **caractérisé par le fait que** le dispositif au moins unique (201) comprend des balises RFID capables d'identifier exclusivement le travailleur.

8. Système (200) selon la réclamation 1, **caractérisé par le fait que** l'unité centrale de traitement (203) est un serveur local ou un serveur en cloud qui héberge une base de données configurée pour le stockage des données liées aux paramètres physiques détectés par le dispositif au moins unique (201) et liées à la fréquence à laquelle les paramètres physiques atteignent les deuxièmes valeurs limites fixées, et un programme logiciel configuré pour l'analyse de ces données.

9. Système (200) selon la réclamation 8, **caractérisé par le fait que** le programme logiciel installé sur l'unité centrale de traitement (203) est configurée pour l'envoi de rapports récurrents à un utilisateur par défaut, ces rapports contenant des données liées aux paramètres physiques sauvegardées sur le dispositif de mémoire réinscriptible (204) et liées à la fréquence à laquelle les paramètres physiques atteignent les deuxièmes valeurs limites fixées concernant chaque travailleur.

10. Méthode (300) d'utilisation du système selon l'une des réclamations précédentes pour détecter et évaluer les facteurs de risque pour la protection de la santé des travailleurs comprenant les étapes :
- (301) de détection des paramètres physiques concernant une activité professionnelle d'un travailleur par le biais d'un dispositif au moins unique pouvant être porté par un travailleur et équipé du capteur au moins unique ;
- (302) de sauvegarde des données liées aux paramètres physiques sur le dispositif de mémoire réinscriptible par le biais d'une unité logique programmable installée sur la carte électronique composée du dispositif au moins unique ;
- (303) d'envoi des données liées aux paramètres physiques à l'unité de contrôle collectant les données grâce à une connexion sans fil de courte portée et de suppression de ces données sur le dispositif de mémoire réinscriptible à la suite de cet envoi ;
- (304) de l'envoi des données liées aux paramètres physiques concernant l'activité professionnelle du travailleur à l'unité centrale de traitement via un réseau LAN d'entreprise ou internet ;
- (305) de traitement des données liées aux paramètres physiques en comparant ces données avec les premières et deuxièmes valeurs limites fixées, ces valeurs limites étant fixées pour chaque paramètre physique évalué ;
**Caractérisée par le fait qu'**elle comprend l'étape (306) de compilation des rapports récurrents à la suite de l'étape (305) de traitement des données liées aux paramètres physiques et d'envoi de ces rapports récurrents à un utilisateur par défaut.
